# EUROPEAN PATENT SPECIFICATION

(11) **EP 3 551 128 B1**
(45) Date of publication and mention of the grant of the patent: **30.12.2020**
(21) Application number: 18720482.1
(22) Date of filing: 04.04.2018
(51) Int. Cl.: A61F 2/01

(54) **EMBOLI-CAPTURING CENTERING DEVICE**
EMBOLI-EINFANGENDE ZENTRIERVORRICHTUNG
DISPOSITIF DE CENTRAGE DE CAPTURE D'EMBOLIES

(30) Priority: 05.04.2017 US 201762481729 P
(43) Date of publication of application: 16.10.2019
(73) Proprietor: Boston Scientific Scimed, Inc., Maple Grove, MN 55113 (US); Mayo Foundation for Medical Education and Research, Rochester, MN 55905 (US)
(72) Inventor: MCGOWAN, Roger W., Ostego, Minnesota 55362 (US); WIEDMANN, Alexander J., Columbia Heights, Minnesota 55421 (US); REINHART, Jennifer, St. Paul, Minnesota 55117 (US); HAVERKOST, Patrick A., Corcoran, Minnesota 55374 (US); OSTROOT, Timothy A., Cokato, Minnesota 55321 (US); SANDHU, Gurpreet S, Rochester, Minnesota 55906 (US); SPOON, Daniel B., Rochester, Minnesota 55906 (US); TEFFT, Brandon J., Rochester, Minnesota 55902 (US)
(74) Representative: Vossius & Partner Patentanwälte Rechtsanwälte mbB
(86) International application number: PCT/US2018/026004
(87) International publication number: WO 2018/187413

(56) References cited:
- WO-A1-2014/169176
- US-A1- 2002 161 394
- US-A1- 2002 173 819
- US-A1- 2010 217 304
- US-A1- 2016 158 006

## Description

### FIELD

The present disclosure relates to devices and methods for cardiovascular treatment and diagnosis. For example, the present disclosure relates to devices and methods by which a catheter can align Itself with a blood flow stream to thereby assist the advancement of a guidewire or other elongate device from the catheter through an orifice of a heart valve, or any other passage In the body against the direction flow, while concurrently providing protection against emboli that may be generated during the course of such a procedure.

### BACKGROUND

Cardiac valvular stenosis is a condition in which the heart's valves are narrowed (stenotic). With valvular stenosis, the tissues forming the valve leaflets become stiffer, narrowing the valve opening, and reducing the amount of blood that can flow through it. If the stenosis is mild, the overall cardiac output remains normal. However, when the valves can become severely stenotic, that can lead to a reduction in cardiac output and impairment of heart function.

For example, mitral valve stenosis is an abnormal narrowing of the mitral valve, resulting in a restriction of the blood flow from the left atrium to the left ventricle. The atrium heart chamber may enlarge as pressure builds up. Blood and fluid may then collect in the lung tissue (pulmonary edema), making it hard to breathe. Mitral valve stenosis can make a person severely short of breath, among other problems.

As another example, aortic valve stenosis occurs when the heart's aortic valve narrows. When the aortic valve is so obstructed, the heart has to work harder to pump blood to the body. Eventually, this extra work limits the amount of blood the heart can pump, and may weaken the heart muscle. The left atrium may enlarge as pressure builds up, and blood and fluid may then collect in the lung tissue (pulmonary edema), making it hard to breathe. Medications can ease symptoms of mild to moderate aortic valve stenosis. However, the only way to treat severe aortic valve stenosis is by surgery.

Therapies to repair or replace the aortic valve include balloon valvuloplasty (valvotomy), surgical aortic valve replacement, and transcatheter aortic valve replacement (TAVR). TAVR Involves replacing the aortic valve with a prosthetic valve that is delivered via the femoral artery (transfemoral) or the left ventricular apex of the heart (transapical).

US 2002/0173819 A1 disloses an apparatus for capturing particulate within a body lumen, comprising a tubular member comprising an inlet and an outlet, the tubular member having a cross-section substantially smaller than a cross-section of a body lumen such that the tubular member may be introduced into a body lumen generally parallel to a natural direction of fluid flow within the body lumen such that the outlet is oriented downstream of the inlet within the body lumen. The apparatus comprises a nozzle disposed within the tubular member and oriented to inject fluid towards the outlet for generating a vacuum at the inlet and a filter element on the inlet. The filter element comprises a plurality of openings having a predetermined maximum size for preventing particulate larger than the maximum size from entering the inlet of the tubular member.

A step that can be challenging when performing a TAVR, valvuloplasty, or hemodynamic study on a stenotic aortic valve is to gain access to the left ventricle through retrograde crossing of a severely stenotic aortic valve with a guidewire, catheter, or other elongate medical device. The valve is also often highly calcified. The current practice can involve repeatedly probing of the stenotic valve with the guide wire until the orifice is penetrated. Prolonged probing can increase the risk of dislodging small amounts of calcified debris and atheroma from the valve surface and can lead to strokes.

### SUMMARY

This disclosure provides devices, systems and methods for the treatment of heart conditions such as valvular stenosis. For example, this disclosure provides devices, systems and methods by which a guide catheter can align itself with a blood flow stream to thereby assist with the advancement of guidewire or other elongate device from the guide catheter, against the direction of blood flow, through a heart valve or other orifice, while at the same time decreasing the risks of unintended embolization arising from the procedure.

The devices and methods provided herein are adapted to save time and expense during cardiac catheterization procedures by aligning a guide catheter with a valve orifice capture emboli that may be released during the course of such procedures. Consequently, the devices and methods provided herein may, for example, reduce overall costs of such procedures, reduce radiation exposure for patients and physicians during such procedures, and reduce the risk of thromboembolic strokes during such procedures. The devices and methods can be used for TAVR procedures as well as for other surgical procedures and diagnostic investigations where there is a need to cross an aortic valve. In addition to treating aortic stenosis, the devices and methods provided herein have applications for perivalvular mitral valve leaks and any other fistulas and openings where there is a need to find a fluid flow and to cross against the fluid flow.

In some general aspects, this disclosure features self-expanding emboli-capturing centering devices for centering a medical instrument in a conduit within a patient. The emboli-capturing centering devices comprise: (a) an elongate shaft; (b) a self-expanding centering member having (i) a flared distal portion having open distal end, (ii) a proximal portion that is attached to the elongate shaft, (iii) an outer surface, and (iv) an inner surface forming a central lumen having a flared distal portion, the centering member being configured such that a fluid flowing in a distal-to-proximal direction that is received by the open distal end of the centering member flows proximally along the flared distal portion of the lumen before exiting the central lumen through one or more openings in the proximal portion of the centering member; and (c) a filter material configured to filter the fluid received by the open distal end of the centering member.

In some embodiments, which may be used in conjunction with the above aspects, the centering member may comprise a flared frame and a covering material disposed over a distal portion of the flared frame, the covering material configured to block passage of blood through the covering material.

In some embodiments, which may be used in conjunction with any of the above aspects and embodiments, the flared frame may comprise a plurality of petal-shaped frame segments.

In some embodiments, which may be used in conjunction with any of the above aspects and embodiments, the filter material may be positioned over the one or more openings in the proximal portion of the centering member.

In some embodiments, which may be used in conjunction with any of the above aspects and embodiments, the filter material is positioned downstream of the one or more openings in the proximal portion of the centering member.

In some embodiments, which may be used in conjunction with any of the above aspects and embodiments, the filter material may be positioned downstream of the one or more openings in the proximal portion of the centering member, and the filter material may extend from a distal end of the centering member, fold over and proximally wrap around the outer surface of the centering member, and attach to the device at a position proximal to the one or more openings in the proximal portion of the centering member. In certain of these embodiments, the centering member may comprise a flared frame and a covering material disposed over a distal portion of the flared frame that is configured to block passage of blood through the covering material in which case, for example, the filter material may be an extension of the covering material (e.g., where pores are formed in the covering material) or the filter material may be a material that is distinct from the covering material.

In some embodiments, which may be used in conjunction with any of the above aspects and embodiments, the filter material may positioned downstream of the one or more openings in the proximal portion of the centering member, and the device may comprise one or more filter pouches having one or more mouths that are attached to the centering member and capture the fluid exiting the one or more openings in the proximal portion of the centering member. In certain of these embodiments, the one or more filter pouches may be attached to a tether, the one or more filter pouches may be attached to a member that slides along an axis of the elongate shaft, or the one or more filter pouches may be attached to the elongate shaft.

In some embodiments, which may be used in conjunction with any of the above aspects and embodiments, the filter material may be positioned downstream of the one or more openings in the proximal portion of the centering member, and the filter material may be supported by an expandable loop structure that self-expands along with the centering member, has a diameter that is greater than that of the centering member, and is supported at a longitudinal position that is approximately the same as that of the open distal end of the centering member. In some of these embodiments, the filter material may in the form of a filter bag having a mouth that attaches to the loop and through which the elongate shaft passes at a position proximal to the centering member. In some of these embodiments, for example, (a) the expandable loop structure may be supported by one or more structural members that extend between the expandable loop structure and the distal end of the centering member or (b) the expandable loop structure may be supported by one or more structural members that extend between the expandable loop structure and the elongate shaft.

In some embodiments, which may be used in conjunction with any of the above aspects and embodiments, the filter material may be positioned downstream of the one or more openings in the proximal portion of the centering member, and the filter material may be attached to an expanding frame that is attached to the elongate shaft at a positon proximal to the centering member. In certain of these embodiments, the expanding frame may be of a greater diameter than the centering member.

In other general aspects, this disclosure features systems for treating a human patient. The systems may comprise (a) a self-expanding emboli-capturing centering device in accordance with any of the above aspects and embodiments, (b) a guide catheter having a lumen, and (c) a guide wire, wherein the self-expanding emboli-capturing centering device is configured to be compressed into a low-profile configuration for advancement through the lumen of the guide catheter and to self-expand to an expanded configuration when expelled from the lumen of the guide catheter, and wherein the guide wire is configured to be advanced through a lumen of the elongate shaft of the self-expanding emboli-capturing centering device.

In other general aspects, this disclosure features methods for treating or diagnosing a human patient using such systems. The methods may comprise (a) inserting the guide catheter into a patient and directing the guide catheter to a target site within the patient; (c) causing the self-expanding emboli-capturing centering device to emerge from a distal end of the lumen of the guide catheter, wherein the self-expanding emboli-capturing centering device reconfigures from a low-profile configuration to an expanded configuration when the self-expanding emboli-capturing centering device emerges from the guide catheter lumen; and (d) causing the guidewire to emerge from a distal end of the lumen of the elongate shaft of the self-expanding emboli-capturing centering device. Such methods may be used, for example, in any procedure where it is desirable to center the centering device on a source of fluid flow and to cause the guidewire to emerge from the centering device and to cross against the fluid flow, while at the same time providing a mechanism for capturing unintended emboli arising from the procedure.

Details of various aspects and embodiments of the invention are set forth in the description to follow and in the accompanying drawings. Other features and advantages of the invention will be apparent from the description and drawings, and from the claims. The present invention is defined by the claims.

### BRIEF DESCRIPTION OF THE DRAWINGS

FIG. 1 is a schematic diagram of a human heart shown in partial cross-section undergoing a catheterization using an emboli-capturing centering device to transmit a guidewire through an aortic valve orifice, in accordance with some embodiments provided herein.
FIG. 2 is a schematic illustration showing a stenosed aortic valve and the flow gradients downstream of the same, which provide enhanced centering forces, relative to a normal aortic valve, for an emboli-capturing centering device in accordance with the present disclosure.
FIG. 3 is a schematic illustration showing a normal aortic valve and the flow gradients downstream of the same, which provide reduced centering forces, relative to a stenosed aortic valve, for an emboli-capturing centering device in accordance with the present disclosure.
FIG. 4 is a schematic illustration of an emboli-capturing centering device in accordance with an embodiment of the present disclosure.
FIG. 5 is a schematic illustration of an emboli-capturing centering device in accordance with another embodiment of the present disclosure.
FIGS. 6A-6C are schematic illustrations of emboli-capturing centering devices in accordance with three embodiments of the present disclosure.
FIG. 7-9 are schematic illustrations of emboli-capturing centering devices in accordance with three further embodiments of the present disclosure.

### DETAILED DESCRIPTION

This disclosure provides devices and methods by which a catheter (e.g., a guide catheter such as a 6 French guide catheter) can align itself with a blood flow stream flowing from an orifice to thereby help direct a guidewire or other elongate device transmitted from the guide catheter through the orifice, while at the same time capturing emboli that may be generated during the course of such procedures.

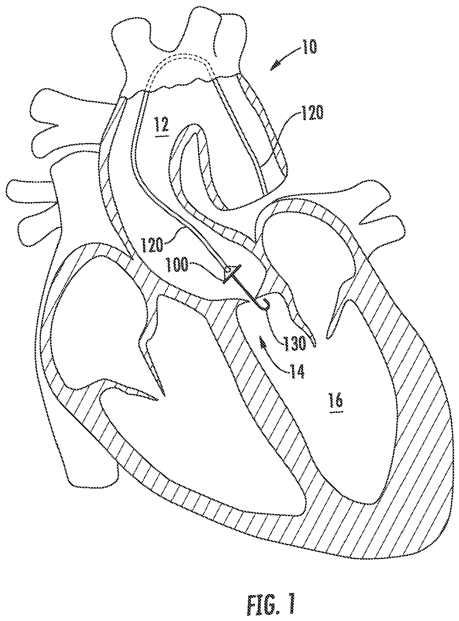

With reference to FIG. 1, a schematic diagram is provided of a human heart **10** shown in partial cross-section undergoing a catheterization using a guide catheter **120**. The guide catheter **120** is depicted in an aortic arch **12** for the purpose of transmitting a guidewire **130** through the orifice of an aortic valve **14.** The blood flow in this region of heart **10** is from a left ventricle **16** to the aortic arch **12.** Therefore, the guide catheter **120** is attempting to insert the guidewire **130** upstream against the blood flowing from the left ventricle **16** to the aortic arch **12.**

The process of crossing a heart valve using a guidewire is performed as a step in various heart treatment and diagnostic procedures. For example, TAVR procedures, valvuloplasties, hemodynamic studies on a stenotic valve, left ventricular ablation, and other types of procedures that involve the placement of a guidewire through the orifice of a heart valve. In addition to aortic valve procedures, other applications involving the placement of a guidewire through an orifice include perivalvular mitral valve leak treatment procedures (or perivalvular aortic valve leak) and other treatment procedures involving fistulas at any site in the human heart or body.

The aortic valve **14** can be approached by the guide catheter **120** via the aortic arch **12.** In some cases, the guide catheter **120** can be percutaneously inserted in a femoral artery of a patient, and directed to the patient's aorta. From the aorta, the guide catheter **120** can be directed to the aortic arch **12.** In other cases, the aortic arch **12** can be accessed by the guide catheter **120** via the patient's radial artery. Other aortic arch **12** access techniques are also envisioned. While the guide catheter **120** is generally linear at its distal end portion in the depicted embodiment, in some embodiments the distal end portion of the guide catheter **120** may be angled (e.g., having a terminal angle). With the distal tip of the guide catheter **120** in a position superior to the aortic valve **14,** the guidewire **130** can be ejected. The purpose of ejecting the guidewire **130** is to insert the guidewire **130** through the orifice of the aortic valve **14.**

The apparatus of FIG. 1 further includes an emboli-capturing centering device **100**. In some embodiments, the emboli-capturing centering device **100** is collapsible to a low-profile delivery configuration and contained within the guide catheter **120** during the insertion of the guide catheter **120** into and within the patient as described herein. When the distal tip of the guide catheter **120** reaches the target site, such as the position superior to the aortic valve **14** as shown, the emboli-capturing centering device **100** can be advanced from the guide catheter **120.** In various embodiments, the emboli-capturing centering device **100** is self-expanding such that the emboli-capturing centering device **100** reconfigures itself from the low-profile delivery configuration to an expanded configuration as shown and includes a centering member having a flared shape. In some embodiments, the guidewire **130** is ejected through a lumen of the emboli-capturing centering device **100**.

When the emboli-capturing centering device **100** is located in a fluid flow path, the shape of the centering member of the emboli-capturing centering device **100** causes the emboli-capturing centering device **100** to center itself in the fluid flow path. As will be described further herein, the flared shape of the centering member of the emboli-capturing centering device **100** is configured to receive or catch a jet flow of fluid (e.g., blood flowing through the orifice of the aortic valve **14** in this example).

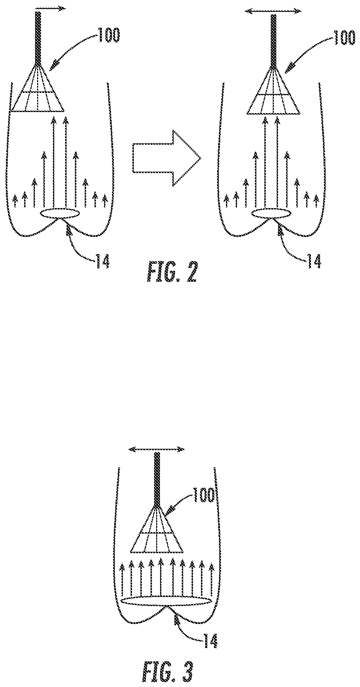

As better seen from FIG. 2, when jet flow of blood flowing from the orifice of the aortic valve **14** contacts an interior side surface of emboli-capturing centering device **100**, the impact force delivered by the jet flow of blood drives the emboli-capturing centering device **100** laterally toward the jet flow so that the jet flow of fluid is caught at or near to the center of the emboli-capturing centering device **100**. The amplified effect of having a narrow jet of fluid flow, for example, emanating from a stenosed aortic valve (because the orifice of the stenosed valve is smaller for the same amount of fluid as a healthy valve), as illustrated schematically in FIG. 2, can be seen by comparison to the effect of a broad region of fluid flow, for example, emanating from a healthy aortic valve, as illustrated schematically in FIG. 3.

Since the emboli-capturing centering device **100** is located at the distal end of the guide catheter **120,** when the emboli-capturing centering device **100** is centered on the fluid flow that is coming from the orifice of the aortic valve **14,** the guide catheter **120** is also centered on such fluid flow. In this manner, the emboli-capturing centering device **100** causes the longitudinal axis of the guide catheter **120** to be in alignment with the orifice of the aortic valve **14.** Therefore, when a guidewire **130** is ejected from guide catheter **120,** the guidewire **130** is more closely aligned to a true center of the orifice of the aortic valve **14,** thereby improving crossing of the guidewire **130** through the aortic valve **14.**

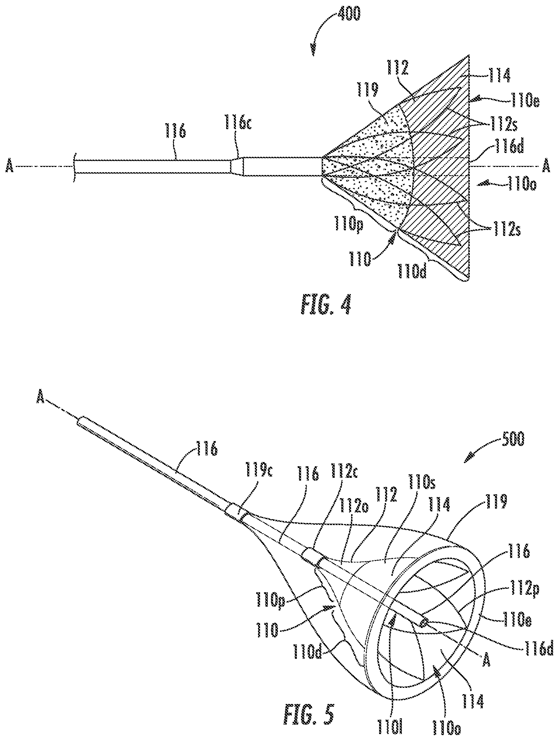

A more detailed view of an emboli-capturing centering device **400** having axis **A** is shown schematically in FIG. 4, in accordance with an embodiment of the present disclosure. The emboli-capturing centering device **400** includes (a) an elongate shaft **116** having a longitudinal axis **A,** a distal end **116d**, and a lumen that is configured to transmit a guidewire, catheter, or other elongate device therethrough and (b) a flared centering member **110** having (i) a proximal portion **110p** that is attached to the elongate shaft **116** and (ii) a distal portion **110d** having an open distal end **110o**, (iii) and a central axial lumen whose diameter decreases in a distal-to-proximal direction in at least the distal portion **110d** of the centering member **110**. In the particular embodiment shown, the centering member **110** includes a flared frame **112,** formed from one or more filamentous elements. A covering material **114,** which blocks passage of a fluid such as blood (e.g., formed from a non-porous material), covers the frame **112** in the distal portion **110d** of the centering member **110**. A filter material **119,** which has openings (e.g., pores having a pore size within a range of 50-200 microns, among other possible sizes) that allow the passage of blood, except for particulates in the blood having a certain minimal size, covers the frame **112** in the proximal portion **110p** of the centering member **110.**

Because the covering material 114 blocks the flow of fluid, fluid that enters the central lumen of the centering member **110** via the open distal end **110o** remains in the central lumen over the length of the distal portion **110d** of the centering member **110** until reaching the proximal portion **110p** of the centering member **110,** whereupon the fluid is able to pass through openings in the filter material **119,** thereby escaping from the axial lumen, and continuing to flow downstream. As a result of this design, fluid entering the axial lumen of the centering member is filtered before continuing downstream. Also as a result of the design, the centering member **110** will tend to self-center itself with a jet of fluid flowing into the axial lumen. Hence, when an emboli-capturing centering device **100** in accordance with the present disclosure is used in conjunction with a guide catheter **120** (see, e.g., FIG. 1), the centering member **110** of the emboli-capturing centering device **100** can center the catheter **120** in relation to blood flow through a narrow orifice, such as blood flow through the orifice of aortic valve **14.** When the catheter **120** is centered in relation to an aortic valve **14,** guidewire **130** can be advanced with an increased likelihood of successful passage through the orifice of the aortic valve **14.** Moreover, blood passing through the centering member **110** is filtered to remove any particulates from the blood over a certain size (e.g., emboli may be captured by a filter material having pores ranging from 50-200 microns in size, among other possible pore sizes), including emboli released during passage of the guidewire **130** through the orifice.

Even assuming that a crossing procedure is successful with relatively few attempts, for example, because the orifice of an aortic valve **14** is crossed by a guidewire **130** on a first crossing attempt, there is still a clinical risk that emboli can become dislodged from the valve and travel downstream. This is especially problematic in the aortic arch **12,** because there is an increased risk of stroke due to the close proximity of the aortic arch **12** to the carotid arteries. Moreover, candidates for TAVR will have a clinical case of aortic valve stenosis, which is often accompanied by significant calcification of the aortic valve **14,** leading to increased clinical risk of dislodgment of calcified emboli from the aortic valve **14.** By employing a filter material **119** in the centering member **110**, emboli larger than the openings in the filter material **119** will be captured, reducing the risk of stroke.

A range of different diameters of the distal end **110e** of the centering member **110** are envisioned, so as to suit different usage variations and body sizes. For example, in some embodiments the distal end **110e** of the centering member **110** ranges between about 5 to 40 millimeters in diameter, about 10 to 35 millimeters in diameter, about 15 to 30 millimeters in diameter, or about 20 to 25 millimeters in diameter. Other centering member **110** diameters of the distal end **110e** are also contemplated.

While the centering member **110** depicted in FIG. 4 and elsewhere herein has a substantially frusto-conical geometry, a range of flared geometries may be employed for the centering member **110** in addition to frusto-conical geometry, including a bell-shaped geometry, a frusto-pyramidal geometry (e.g., having 3, 4, 5, 6, 7, 8, 9, 10 sides or more), and so forth.

The flared frame **112,** for use in conjunction with the present disclosure, may be formed from a single piece of material (e.g., in the form of a single-filament construction or a laser-cut tube that is expanded into to a desired flared shape, which is heat-set to make the flared shape the natural configuration) or formed from a plurality of frame segments **112s** (e.g., thin strands, such as wires, filaments, ribbons, and so forth) that are shaped and held together, for instance by welding, adhesives, mechanical means, and so forth, to create the flared frame **112.**

The frame **112** may be formed from various metals and metal alloys, including nitinol (NiTi alloy) (e.g., superelastic nitinol), stainless steel, titanium, and Elgiloy (Co-Cr-Ni Alloy), among others. The frame **112** may also be formed from various polymers.

In general, the frame **112** is collapsible to fit within the lumen of a catheter. The frame **112** can radially self-expand to a flared unconstrained configuration as shown upon deployment from the catheter. In such an example, the frame **112** may be comprised of a shape-memory material, for example, a shape memory alloy such as super-elastic nitinol (NiTi) material or a shape memory polymer.

In the specific embodiment shown in FIG. 4 (as well as FIGS. 5-9 described below), the frame **112** is constructed from a series of petal shaped frame segments **112s** (e.g., three, four, five, six, seven, eight, nine, ten, or more petal shaped frame segments **112s**) positioned around the device axis **A.** The petal shaped frame segments **112s** are configured to collapse for containment within a guide catheter **120.** Upon emergence from the guide catheter **120,** the petal shaped frame segments **112s** are configured to open up to create an overall flared shape, in particular, a generally frusto-conical shape as shown, a bell shape or other funnel shape, among others. In various embodiments, the petal shaped frame segments **112s** are constructed of a shape-memory material that facilitates the collapsibility and expandability of the petals. The petal shaped frame segments **112s** are formed from thin strands (e.g., wires, filaments, ribbons, etc.) of a suitable material. In various embodiments, the petal shaped frame segments **112s** overlap with each other upon expansion. In such embodiments, (a) in the distal portion **110d** where the covering material **114** covers the frame **112,** the covering material **114** may cover roughly triangular openings and (b) in the proximal portion **110p**, where the filter material **119** covers the frame **112,** the filter material **119** may cover roughly triangular and quadrilateral openings between the filaments forming the petal shaped frame segments **112s**.

In some embodiments, the frame **112** may include one or more visualization markers (not shown), such as radiopaque markers, bands, or radiopaque filler materials. The radiopaque markers can assist a clinician with in situ radiographic visualization of the centering member **110** so that the clinician can orient the device as desired in relation to the anatomy of the patient.

The covering material **114** employed in the embodiment shown in FIG. 4, as well as other embodiments described herein (see, e.g., FIGS. 5-9 described below), may be made of any flexible, biocompatible material capable of acting as a barrier to fluid flow (e.g., blood emerging from the orifice of a heart valve). Such covering materials can include, but are not limited to polymeric films and fabrics including polyesters such as polyethylene terephthalate, fluoropolymers such as polytetrafluoroethylene (PTFE) and expanded polytetrafluoroethylene (ePTFE), polyamides, including nylon, polyether block amides (e.g., PEBAX), chemical vapor deposited polyxylylene polymers (parylenes) including poly(p-xylylene) polymers, polysiloxanes including silicone, polyurethanes, including thermoplastic polyurethanes (TPU), such as polyether polyurethanes, polyisobutylene-polyurethanes, polyurethane-silicone copolymers, poly(vinylidene fluoride-hexafluoropropylene) (PVDF-HFP), polyisobutylene copolymers including polyisobutylene-polystyrene block copolymers such as polystyrene-polyisobutylene-polystyrene triblock copolymers (SIBS), as well as metallic films, or combinations of the foregoing materials.

The filter material **119** employed in the embodiment shown in FIG. 4, as well as other embodiments described herein (see, e.g., FIGS. 5-9 described below), may be made of any flexible, biocompatible material which can be formed or processed in a manner so as to render the material porous, thereby allowing for the filtration of particulates. Pore sizes suitable for capturing potentially harmful emboli may range, for example, from 50-200 microns, among other possible pore sizes. For example, in some embodiments, the filter material **119** may be comprised of ePTFE or another polymer, and may have ∼100um laser-drilled pores with ∼50% porosity. Examples of specific materials for use as filter materials include polymers selected from those set forth immediately above for use as covering materials.

In FIG. 4, as well as other embodiments described herein (see, e.g., FIGS. 5-9 described below), the covering material **114** and the filter material **119** may be attached to structural members, including the frame **112** and the segments **112s,** using a variety of techniques. For example, in some embodiments, the covering material **114** and/or the filter material **119** may be sewn to structural members. In some embodiments, the covering material **114** and/or the filter material **119** may be glued to structural members. In some embodiments, structural members may be sandwiched between layers of the covering material **114,** sandwiched between layers of the filter material **119,** or sandwiched between a layer of the covering material **114** and a layer of the filter material **119.** In some embodiments, a combination of such attachment methods are used.

It is noted that, while the centering member **110** depicted in FIG. 4 and elsewhere herein (e.g., FIGS. 5-9) is formed from the frame **112** and the covering material **114,** the centering member **110** need not be so constructed. For example, in some embodiments, the entire centering member **110** may be constructed from a flexible self-expanding material, such as an elastomeric material, which can be collapsed into a tubular structure for delivery, afterwards spring open to form a structure that has an axial lumen that is flared at its distal end to receive fluid and has apertures at its proximal end to allow that fluid to exit. In some such embodiments, the centering member **110** may include pleats and/or hinges that facilitate the collapsibility and expandability of the device.

Although the filter material **119** is employed in the proximal portion **110p** of the centering. member **110** in FIG. 4, in other embodiments, the filter material **119** may be placed in other portions of the emboli-capturing centering device **100**.

One embodiment of such an emboli-capturing centering device **500** is schematically illustrated FIG. 5. Analogous to FIG. 4, the emboli-capturing centering device **500** of FIG. 5 includes an elongate shaft **116** having a distal end **116d**, a flared centering member **110** having a proximal portion **110p** that is attached to the elongate shaft **116** and a distal portion **110d** having an open distal end **110ο**. More specifically, in the embodiment shown, the centering member **110** includes a flared frame **112** which is attached to the elongate shaft **116** by a collar **112c,** and a covering material **114,** which covers the frame **112** in the distal portion **110d** of the centering member **110**. The centering member **110** also includes a central axial lumen **110l** whose diameter decreases in a distal-to-proximal direction, at least within the distal portion **110d** of the centering member **110**.

Unlike Fig. 4, in the emboli-capturing centering device **500** of FIG. 5, the filter material **119** extends from a distal end **110e** of the centering member **110**, folding over and proximally wrapping around an outer surface **110s** of the centering member **110**, and attaching to the elongate shaft **116** at a position proximal to the openings between the elements of the frame **112** in the proximal portion **110p** of the centering member **110**. As elsewhere herein, the filter material **119** has openings that allow the passage of blood except for particulates in the blood having a certain minimal size (e.g., particulates captured by a filter material having pores ranging from 50-200 microns in size, among other possible pore sizes). In some embodiments, the filter material **119** may be an extension of the covering material **114,** in which case openings may be formed in the material in those areas corresponding to the filter material **119** in order to give the material particle-filtering properties. In some embodiments, the filter material **119** may be formed of a different material than that the covering material **114** and may be attached, at the distal end **110e** of the centering member **110**, to the frame **112,** to the covering material **114,** or both.

Consequently, in the device of FIG. 5, fluid flowing in a distal-to-proximal direction that is received by the open distal end **110o** of the centering member **110** flows proximally along a portion of the central lumen **110l** in the distal portion **110d** of the centering member **110** before exiting the central lumen **110l** through the openings **112o** between the elements of the frame **112** in the proximal portion **110p** of the centering member **110.** After passing through the openings **110ο,** the fluid passes through the filter material **119,** thereby removing emboli of a certain minimal size that may be present (e.g., emboli captured by a filter material having pores ranging from 50-200 microns in size, among other possible pore sizes). It is noted that, under the same flow conditions and using the same filter material **119,** fluid flow through the centering member **110** of FIG. 5 may be greater than fluid flow through the centering member **110** of FIG. 4, due to the substantially greater area of the filter material **119** in FIG. 5 relative to FIG. 4, through which the fluid can flow.

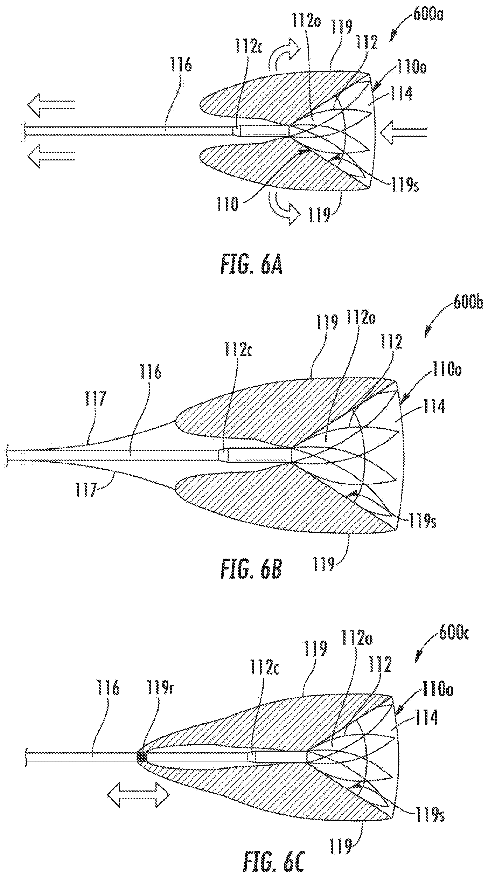

Various additional configurations for placement of the filter material **119** are also envisioned. For example, with reference to FIG. 6A, an emboli-capturing centering device **600a** is schematically illustrated which includes an elongate shaft **116,** a flared centering member **110** having a proximal portion that is attached to the elongate shaft **116** and a distal portion having an open distal end. The flared centering device **600a** includes a central lumen that tapers from one diameter at the open distal end of the centering device **600a** to a lesser diameter at a position proximal to the open distal end of the centering device **600a** and, in the embodiment shown, includes a flared frame **112,** which is attached to the elongate shaft **116,** terminating at collar **112c,** which marks a transition between the flared frame **112** and the elongate shaft **116.** The most proximal portion of the flared frame **112** may lie parallel to the elongate shaft 116. In some embodiments, a retention collar may be provided, for example, which extends over at least some of the length of, and in some embodiments over all of the length of, the proximal portion of the flared frame **112** that lies parallel to the elongate shaft **116,** thereby enhancing retention of the proximal end of the flared frame **112** to the elongate shaft **116.** The flared centering device **600a** further includes a covering material **114,** which covers the frame **112** in the distal portion of the centering member **110**.

The filter material in Fig. 6A is in the form of one or more filter pouches **119** that are attached to the centering member **110** around the entire circumference of the centering member **110** such that fluid flowing in a distal-to-proximal direction that is received by the open distal end **110o** of the centering member **110** flows proximally along a portion of the central lumen of the centering member **110** before exiting the central lumen of the centering member **110** through the openings **112o** between the elements of the frame **112** and into the one or more filter pouches **119,** causing the one or more pouches **119** to billow outward to a degree, as shown. Emboli of a certain minimal size that may be present (e.g., emboli that are captured by a filter material having pores ranging from 50-200 microns in size, among other possible pore sizes) are thereby removed upon passing through the filter material forming the filter pouches **119.** It is noted that the one or more filter pouches **119** in FIG. 6A are shown in cross-section, with the cross-hatching representing the hollow interior of the filter pouches **119.** In an embodiment where the device **600a** contains a single filter pouch **119,** the pouch **119** would be in the form of a hollow ring or "donut", extending 360° about the axis of the device **600a** and having a slot **119s** that also extends 360° about the axis of the device **600a** and provides fluid communication between the Interior or the pouch **119** and the proximal portion of the centering member **110**, such that all fluid flowing exiting through the openings **112o** between the elements of the frame **112** in the proximal portion of the centering member **110** will pass through the filter pouch **119** before exiting the device **600a**.

While the proximal end(s) of the one or more filter pouches **119** in Fig. 6A float(s) freely, in FIGS. 6B and 6C, the proximal ends of the of the one or more filter pouches **119** are, to a degree, constrained. In this regard, with reference to FIG. 6B, an emboli-capturing centering device **600b** is shown, which is analogous to that of FIG. 6A, except one or more tethers **117** (e.g., in the form of one or more filaments) is/are attached to a proximal end of each of the one or more filter pouches **119.** During a medical procedure, the tether(s) **117** extend proximally through the guide catheter (not shown) to an exterior of the body where the tether(s) **117** can be manipulated by a clinician. In this way, after a medical procedure, the one or more filter pouches **119** with captured particulate could enter the guide catheter before the centering member **110**, pulled in by the tether(s) **117.**

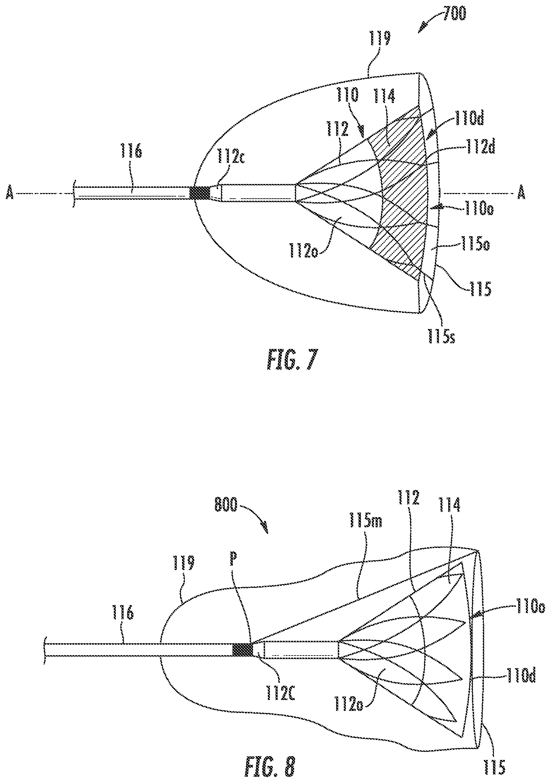

With reference to FIG. 6C, an emboli-capturing centering device **600c** is shown, which is analogous to that of FIG. 6A, except that a proximal end of each of the one or more filter pouches **119** is attached to a slidable member **119r** (in the form of a ring in the embodiment shown) that is slidable along a portion of the length of the elongate shaft **116.** During a medical procedure, the slidable member **119r** allows a guide catheter (not shown) to slide up to a proximal end of the centering member **110** (i.e., up to the collar **112c** in the embodiment shown), thereby supporting the centering member **110**. Referring now to FIG. 7, an emboli-capturing centering device **700** is shown, which includes an elongate shaft **116,** a flared centering member **110** having a proximal portion that is attached to the elongate shaft **116** and a distal portion with an open distal end **110o**. The flared centering member **110** includes a central lumen that tapers from one diameter at the open distal end of the centering member **110** to a lesser diameter at a position proximal to the open distal end of the centering member **110** and, in the embodiment shown, includes (a) a flared frame **112,** which is attached to the elongate shaft **116** by a collar **112c** and (b) a region of covering material **114** covering the frame **112** in a distal portion **110d** of the centering member **110.**

The filter material in Fig. 7 is in the form of a single filter bag **119** (shown in cross-section) having a mouth that is attached to an expandable loop structure **115,** which has an expanded diameter that is greater than the expanded diameter of the distal end **112d** of the frame **112** (and thus has an expanded diameter that is greater than the expanded diameter of the centering member **110**). The expandable loop structure **115** is supported, at a longitudinal position along an axis of the device **A** that is approximately the same as that of the distal end **110d** of the centering member **110**, by a series of support members **115s** that extend between the expandable loop structure **115** and the distal end **112d** of the frame **112** (at the tips of petal shaped frame segments forming frame 112 in the embodiment shown). The expandable loop structure **115** and the support members **115s** in these embodiments are an extension of the frame **112** and, thus can be created in a single piece or a separate pieces, as desired. In this design, fluid flowing in a distal-to-proximal direction that is received by the open distal end **110o** of the centering member **110** will flow proximally along a portion of the central lumen of the centering member **110** before exiting the central lumen of the centering member **110** through the openings **112o** of the frame **112** and into the filter bag **119.** In addition, fluid flowing in a distal-to-proximal direction that is received in the openings **115o** between the loop structure **115** and the open distal end **110o** of the centering member **110** will flow proximally and directly into the filter bag **119.** Emboli of a certain minimal size that may happen to be present (e.g., emboli that are captured by a filter material having pores ranging from 50-200 microns in size, among other possible pore sizes) will be removed upon passing through the filter material forming the filter bag **119.**

An embodiment of an emboli-capturing centering device **800** illustrated in FIG. 8 is similar to that of FIG. 7, except that the expandable loop structure **115** is supported, at a longitudinal position that is approximately the same as that of the distal end **110d** of the centering member **110**, by a support member **115m** that extends from the expandable loop structure **115** to an attachment point **P** which can be on the elongate shaft **116** as shown or, for example, on a proximal portion of the centering member 110. The expandable loop 115 may be connected to the elongate shaft 116, for example, via an interface where one or more support members **115m** are adjoined to one or more attachment points P by a stent-like structure (e.g., a precision cut tube interface structure, a slotted tube type structure, etc.), by a metal collar, such as a marker band or a stainless steel hypo-tube, by a wrap of material (e.g., a metallic or polymeric wire, ribbon or thread), by an adhesive joint, and so forth. As with Fig. 7, (a) fluid flowing in a distal-to-proximal direction that is received by the open distal end **110o** of the centering member **110** will flow proximally along a portion of the central lumen of the centering member **110** before exiting the central lumen of the centering member **110** through the openings **112o** and into the filter bag **119** and (b) fluid flowing in a distal-to-proximal direction that is received between the loop structure **115** and the open distal end **110o** of the centering member **110** will flow proximally and directly into the filter bag **119.** Emboli of a certain minimal size that may happen to be present (e.g., emboli that are captured by a filter material having pores ranging from 50-200 microns in size, among other possible pore sizes) will be removed upon passing through the filter material forming the filter bag **119.**

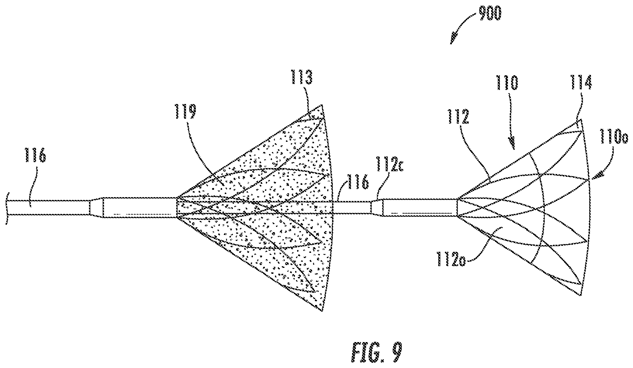

Referring now to FIG. 9, an emboli-capturing centering device **900** is shown, which includes an elongate shaft **116,** a flared centering member **110** having a proximal portion that is attached to the elongate shaft **116** and a distal portion with an open distal end **110ο**. As in prior embodiments, the centering device **900,** includes a flared frame **112** which is attached to the elongate shaft **116** and a region of covering material **114** covering the frame **112** in a distal portion of the centering member **110**. The filter material **119** in Fig. 9, however, is attached to a frame **113** lying proximal the frame **112.** Although the frame **113** is of a design analogous to that of frame **112** in Fig. 9, this is by no means required. In certain embodiments, a diameter of the frame **113** may be greater than a diameter of the frame **112,** in order to enhance embolic protection provided by the device **900.** For example, the diameter of the frame **113** may range from 50% to 200% of diameter of the frame **112.** In this design, fluid flowing in a distal-to-proximal direction that is received by the open distal end **110o** of the centering member **110** will flow proximally along a portion of the central lumen of the centering member **110** before exiting the central lumen of the centering member **110** through the openings **112o.** As the fluid exiting the openings **112o** continues downstream, at least a portion of this fluid will flow through the filter material **119,** capturing emboli of a certain minimal size (e.g., emboli that are captured by a filter material having pores ranging from 50-200 microns in size, among other possible pore sizes) that may happen to be present.

It should be understood that one or more of the features described in reference to any one embodiment described herein may be combined with one or more of the features of other embodiments provided herein. That is, various features described herein can be mixed and matched to create hybrid designs, and such hybrid designs are within the scope of this disclosure.

As previously indicated, devices in accordance with the present disclosure are useful in advancing a guidewire or other elongate device from a catheter through an orifice (such as a heart valve) where a fluid is flowing in a counter direction to the direction the guidewire is being advanced, while at the same time filtering emboli that may be generated during the procedure. In a typical procedure, a guide catheter is inserted into a patient by a clinician. In some cases, the insertion may be percutaneous. In some cases, the insertion may be through a natural body orifice or channel. A self-expanding emboli-capturing centering device in accordance with the present disclosure can be contained in a low profile within a lumen of the catheter, and the guidewire can be advanced through a lumen in the self-expanding emboli-capturing centering device.

In use, a distal end of the guide catheter is directed to the target site within the body of the patient. Visualization systems such as x-ray fluoroscopy, MRI, or ultrasound can be utilized to assist the clinician with directing the guide catheter within the patient as desired. Once the distal end of the guide catheter is located at a desired site within the patient's body, the clinician can deploy the emboli-capturing centering device from the distal end of the guide catheter. As the centering member of the emboli-capturing centering device emerges from the confines of the guide catheter lumen, the centering member can self-expand to a flared shape as described herein. With the self-expanding emboli-capturing centering device supported by the guide catheter in the flow path of a fluid (such as blood flow from an orifice of a heart valve), the centering member will receive at least a portion of the fluid that is flowing toward the distal tip of the guide catheter. The impact of the fluid on the inner surface of the centering member will cause the centering member to move laterally so as to center itself on the fluid flow. When the fluid flow is from an orifice, the lateral movement of the centering member will cause the centering member be centered in relation to the orifice. With the axis of the elongate shaft of the self-expanding emboli-capturing centering device centered on the orifice, the clinician can eject the guidewire from the lumen of the elongate shaft. The guidewire will emerge from the lumen and be in alignment with the orifice. As such, the guidewire can be advanced through the orifice by the clinician.

## Claims

1. A self-expanding emboli-capturing centering device (100; 400; 500; 600a; 600b; 600c;
700; 800; 900) for centering a medical instrument in a conduit within a patient, the self-expanding emboli-capturing centering device (100; 400; 500; 600a; 600b; 600c; 700; 800; 900) comprising:
an elongate shaft (116);
a self-expanding centering member (110) having (i) a flared distal portion (110d) having an open distal end (110ο), (ii) a proximal portion (110p) that is attached to the elongate shaft (116), (iii) an outer surface (110s), and (iv) an inner surface forming a central lumen (110l) having a flared distal portion, the centering member (110) configured such that a fluid flowing in a distal-to-proximal direction that is received by the open distal end (110o) of the centering member (110) flows proximally along the flared distal portion of the lumen (1101) before exiting the central lumen through one or more openings in the proximal portion (110p) of the centering member (110); and
a filter material (119) configured to filter the fluid received by the open distal end (110o) of the centering member (110).

2. The self-expanding emboli-capturing centering device (400; 500; 600a, 600b, 600c; 700; 800; 900) of claim 1, wherein the centering member (110) comprises a flared frame (112) and a covering material (114) disposed over a distal portion of the flared frame (112), the covering material (114) configured to block passage of blood through the covering material (114).

3. The self-expanding emboli-capturing centering device (400; 500; 600a, 600b, 600c; 700; 800; 900) of claim 2, wherein the flared frame (112) comprises a plurality of petal-shaped frame segments (112s).

4. The self-expanding emboli-capturing centering device (400; 500; 600a; 600b; 600c; 700; 800) of any one of claims 1-3, wherein the filter material (119) is positioned over the one or more openings (112o) in the proximal portion (110p) of the centering member (110).

5. The self-expanding emboli-capturing centering device (400; 500; 600a; 600b; 600c; 700; 800) of any one of claims 1-3, wherein the filter material (119) is positioned downstream of the one or more openings (112o) in the proximal portion (110p) of the centering member (110).

6. The self-expanding emboli-capturing centering device (500; 600a; 600b; 600c; 700; 800) of claim 5, wherein the filter material (119) extends from a distal end (110e) of the centering member (110), folds over and proximally wraps around the outer surface (110s) of the centering member (110), and attaches to the device (500; 600a; 600b; 600c; 700; 800) at a position proximal to the one or more openings (112o) in the proximal portion (110p) of the centering member (110).

7. The self-expanding emboli-capturing centering device (500; 600a; 600b; 600c; 700; 800) of claim 6, wherein the centering member (110) comprises a flared frame (112) and a covering material (114) disposed over a distal portion of the flared frame (112), wherein the covering material (114) is configured to block passage of blood through the covering material (114), and (i) wherein the filter material (119) is an extension of the covering material (114) in which pores are formed or (ii) wherein the filter material (119) is a material that is distinct from the covering material (114).

8. The self-expanding emboli-capturing centering device (600a; 600b; 600c) of claim 5, comprising one or more filter pouches (119) having one or more mouths that are attached to the centering member (110) and capture the fluid exiting the one or more openings in the proximal portion (110p) of the centering member (110).

9. The self-expanding emboli-capturing centering device (600b) of claim 8, wherein each of the one or more filter pouches (119) is attached to a tether (117), or wherein each of the one or more filter pouches (119) are attached to a member that slides along an axis of the elongate shaft (116), or wherein each of the one or more filter pouches (119) is attached to the elongate shaft (116).

10. The self-expanding emboli-capturing centering device (700; 800) of claim 5, wherein the filter material (119) is supported by an expandable loop structure (115) that self-expands along with the centering member (110) and has a diameter that is greater than that of the centering member (110) and is supported at a longitudinal position that is approximately the same as that of the open distal end (110o) of the centering member (110).

11. The self-expanding emboli-capturing centering device (700) of claim 10, wherein the expandable loop structure (115) is supported by one or more structural members (115s) that extend between the expandable loop structure (115) and the distal end of the centering member (110), or wherein the expandable loop structure (115) is supported by one or more structural members (115s) that extend between the expandable loop structure (115) and the elongate shaft (116).

12. The self-expanding emboli-capturing centering device (700; 800) of claim 10, wherein the filter material is in the form of a filter bag (119) having a mouth that attaches to the loop (115) and through which the elongate shaft (116) passes at a position proximal to the centering member (110).

13. The self-expanding emboli-capturing centering device (400; 500; 600a; 600b; 600c; 700; 800) of claim 5, wherein the filter material (119) is attached to an expanding frame that is attached to the elongate shaft (116) at a position proximal to the centering member (110).

14. The self-expanding emboli-capturing centering device (400; 500; 600a; 600b; 600c; 700; 800) of claim 13, wherein the expanding frame is of greater diameter than the centering member (110).

15. A system comprising: (a) the self-expanding emboli-capturing centering device (100; 400; 500; 600a; 600b; 600c; 700; 800; 900) of any one of claims 1-14, (b) a guide catheter (120) having a lumen, and (c) a guide wire (130), wherein the self-expanding emboli-capturing centering device (100; 400; 500; 600a; 600b; 600c; 700; 800; 900) is configured to be compressed into a low-profile configuration for advancement through the lumen of the guide catheter (120) and to self-expand to an expanded configuration when expelled from the lumen of the guide catheter (120), and wherein the guide wire (130) is configured to be advanced through a lumen of the elongate shaft (116) of the self-expanding emboli-capturing centering device (100; 400; 500; 600a; 600b; 600c; 700; 800; 900).

## Patentansprüche

1. Selbstexpandierende Emboli einfangende Zentriervorrichtung (100; 400; 500; 600a; 600b; 600c; 700; 800; 900) zum Zentrieren eines medizinischen Instruments in einem Kanal in einem Patienten, wobei die selbstexpandierende Emboli einfangende Zentriervorrichtung (100; 400; 500; 600a; 600b; 600c; 700; 800; 900) aufweist:
einen länglichen Schaft (116);
ein selbstexpandierendes Zentrierteil (110) mit (i) einem aufgeweiteten distalen Abschnitt (110d), der ein offenes distales Ende (110o) hat, (ii) einem proximalen Abschnitt (110p), der am länglichen Schaft (116) angebracht ist, (iii) einer Außenfläche (110s) und (iv) einer Innenfläche, die ein Zentrallumen (110l) mit einem aufgeweiteten distalen Abschnitt bildet, wobei das Zentrierteil (110) so konfiguriert ist, dass ein in Richtung von distal nach proximal fließendes Fluid, das durch das offene distale Ende (110o) des Zentrierteils (110) aufgenommen wird, entlang des aufgeweiteten distalen Abschnitts des Lumens (110l) proximal fließt, bevor es aus dem Zentrallumen über eine oder mehrere Öffnungen im proximalen Abschnitt (110p) des Zentrierteils (110) austritt; und
ein Filtermaterial (119), das so konfiguriert ist, dass es das Fluid filtert, das durch das offene distale Ende (110o) des Zentrierteils (110) aufgenommen wird.

2. Selbstexpandierende Emboli einfangende Zentriervorrichtung (400; 500; 600a; 600b; 600c; 700; 800; 900) nach Anspruch 1, wobei das Zentrierteil (110) einen aufgeweiteten Rahmen (112) und ein Abdeckmaterial (114) aufweist, das über einem distalen Abschnitt des aufgeweiteten Rahmens (112) angeordnet ist, wobei das Abdeckmaterial (114) so konfiguriert ist, dass es den Durchgang von Blut durch das Abdeckmaterial (114) blockiert.

3. Selbstexpandierende Emboli einfangende Zentriervorrichtung (400; 500; 600a, 600b, 600c; 700; 800; 900) nach Anspruch 2, wobei der aufgeweitete Rahmen (112) mehrere blütenblattförmige Rahmensegmente (112s) aufweist.

4. Selbstexpandierende Emboli einfangende Zentriervorrichtung (400; 500; 600a; 600b; 600c; 700; 800) nach einem der Ansprüche 1 bis 3, wobei das Filtermaterial (119) über der einen oder den mehreren Öffnungen (112o) im proximalen Abschnitt (110p) des Zentrierteils (110) positioniert ist.

5. Selbstexpandierende Emboli einfangende Zentriervorrichtung (400; 500; 600a; 600b; 600c; 700; 800) nach einem der Ansprüche 1 bis 3, wobei das Filtermaterial (119) stromabwärts von der einen oder den mehreren Öffnungen (112o) im proximalen Abschnitt (110p) des Zentrierteils (110) positioniert ist.

6. Selbstexpandierende Emboli einfangende Zentriervorrichtung (500; 600a; 600b; 600c; 700; 800) nach Anspruch 5, wobei sich das Filtermaterial (119) von einem distalen Ende (110e) des Zentrierteils (110) erstreckt, umgelegt und um die Außenfläche (110s) des Zentrierteils (110) proximal gewickelt ist sowie an der Vorrichtung (500; 600a; 600b; 600c; 700; 800) an einer Position proximal zu der einen oder den mehreren Öffnungen (112o) im proximalen Abschnitt (110p) des Zentrierteils (110) angebracht ist.

7. Selbstexpandierende Emboli einfangende Zentriervorrichtung (500; 600a; 600b; 600c; 700; 800) nach Anspruch 6, wobei das Zentrierteil (110) einen aufgeweiteten Rahmen (112) und ein Abdeckmaterial (114) aufweist, das über einem distalen Abschnitt des aufgeweiteten Rahmens (112) angeordnet ist, wobei das Abdeckmaterial (114) so konfiguriert ist, dass es den Durchgang von Blut durch das Abdeckmaterial (114) blockiert, und (i) wobei das Filtermaterial (119) eine Verlängerung des Abdeckmaterials (114) ist, in der Poren gebildet sind, oder (ii) wobei das Filtermaterial (119) ein Material ist, das sich vom Abdeckmaterial (114) unterscheidet.

8. Selbstexpandierende Emboli einfangende Zentriervorrichtung (600a; 600b; 600c) nach Anspruch 5, das eine oder mehrere Filtertaschen (119) mit einer oder mehreren Mündungen aufweist, die am Zentrierteil (110) angebracht sind und das Fluid einfangen, das aus der einen oder den mehreren Öffnungen im proximalen Abschnitt (110p) des Zentrierteils (110) austritt.

9. Selbstexpandierende Emboli einfangende Zentriervorrichtung (600b) nach Anspruch 8, wobei die eine oder die mehreren Filtertaschen (119) jeweils an einer Leine (117) angebracht sind oder wobei die eine oder die mehreren Filtertaschen (119) jeweils an einem Teil angebracht sind, das entlang einer Achse des länglichen Schafts (116) gleitet, oder wobei die eine oder die mehreren Filtertaschen (119) jeweils am länglichen Schaft (116) angebracht sind.

10. Selbstexpandierende Emboli einfangende Zentriervorrichtung (700; 800) nach Anspruch 5, wobei das Filtermaterial (119) durch eine expandierbare Schlaufenstruktur (115) gestützt wird, die zusammen mit dem Zentrierteil (110) selbstexpandiert und einen Durchmesser hat, der größer als der des Zentrierteils (110) ist und an einer Längsposition gestützt wird, die etwa die gleiche wie die des offenen distalen Endes (110o) des Zentrierteils (110) ist.

11. Selbstexpandierende Emboli einfangende Zentriervorrichtung (700) nach Anspruch 10, wobei die expandierbare Schlaufenstruktur (115) durch ein oder mehrere Strukturteile (115s) gestützt wird, die sich zwischen der expandierbaren Schlaufenstruktur (115) und dem distalen Ende des Zentrierteils (110) erstrecken, oder wobei die expandierbare Schlaufenstruktur (115) durch ein oder mehrere Strukturteile (115s) gestützt wird, die sich zwischen der expandierbaren Schlaufenstruktur (115) und dem länglichen Schaft (116) erstrecken.

12. Selbstexpandierende Emboli einfangende Zentriervorrichtung (700; 800) nach Anspruch 10, wobei das Filtermaterial die Form eines Filterbeutels (119) mit einer Mündung hat, die an der Schlaufe (115) angebracht ist und durch die der längliche Schaft (116) an einer Position proximal zum Zentrierteil (110) durchläuft.

13. Selbstexpandierende Emboli einfangende Zentriervorrichtung (400; 500; 600a; 600b; 600c; 700; 800) nach Anspruch 5, wobei das Filtermaterial (119) an einem expandierenden Rahmen angebracht ist, der am länglichen Schaft (116) an einer Position proximal zum Zentrierteil (110) angebracht ist.

14. Selbstexpandierende Emboli einfangende Zentriervorrichtung (400; 500; 600a; 600b; 600c; 700; 800) nach Anspruch 13, wobei der expandierende Rahmen einen größeren Durchmesser als das Zentrierteil (110) hat.

15. System, das aufweist: (a) die selbstexpandierende Emboli einfangende Zentriervorrichtung (100; 400; 500; 600a; 600b; 600c; 700; 800; 900) nach einem der Ansprüche 1 bis 14, (b) einen Führungskatheter (120) mit einem Lumen und (c) einen Führungsdraht (130), wobei die selbstexpandierende Emboli einfangende Zentriervorrichtung (100; 400; 500; 600a; 600b; 600c; 700; 800; 900) so konfiguriert ist, dass sie in eine kleine Querschnittskonfiguration zum Vorschieben durch das Lumen des Führungskatheters (120) zusammengedrückt wird und in eine expandierte Konfiguration selbstexpandiert, wenn sie aus dem Lumen des Führungskatheters (120) ausgestoßen wird, und wobei der Führungsdraht (130) so konfiguriert ist, dass er durch ein Lumen des länglichen Schafts (116) der selbstexpandierenden Emboli einfangenden Zentriervorrichtung (100; 400; 500; 600a; 600b; 600c; 700; 800; 900) vorgeschoben wird.

## Revendications

1. Dispositif de centrage auto-expansible de capture d'emboles (100 ; 400 ; 500 ; 600a ; 600b ; 600c ; 700 ; 800 ; 900) pour centrer un instrument chirurgical dans un conduit à l'intérieur d'un patient, le dispositif de centrage auto-expansible de capture d'emboles (100 ; 400 ; 500 ; 600a ; 600b ; 600c ; 700 ; 800 ; 900) comprenant :
un arbre allongé (116) ;
un élément de centrage auto-expansible (110) ayant (i) une partie distale évasée (110d) ayant une extrémité distale ouverte (110o), (ii) une partie proximale (110p) qui est fixée à l'arbre allongé (116), (iii) une surface externe (110s), et (iv) une surface interne formant une lumière centrale (110l) ayant une partie distale évasée, l'élément de centrage (110) étant configuré de sorte qu'un fluide s'écoulant dans une direction distale - proximale qui est reçu par l'extrémité distale ouverte (110o) de l'élément de centrage (110) s'écoule de manière proximale le long de la partie distale évasée de la lumière (110l) avant de sortir de la lumière centrale à travers une ou plusieurs ouvertures dans la partie proximale (110p) de l'élément de centrage (110) ; et
un matériau de filtre (119) configuré pour filtrer le fluide reçu par l'extrémité distale ouverte (110o) de l'élément de centrage (110).

2. Dispositif de centrage auto-expansible de capture d'emboles (400 ; 500 ; 600a ; 600b ; 600c ; 700 ; 800 ; 900) selon la revendication 1, dans lequel l'élément de centrage (110) comprend un bâti évasé (112) et un matériau de recouvrement (114) disposé sur une partie distale du bâti évasé (112), le matériau de recouvrement (114) étant configuré pour bloquer le passage du sang à travers le matériau de recouvrement (114).

3. Dispositif de centrage auto-expansible de capture d'emboles (400 ; 500 ; 600a ; 600b ; 600c ; 700 ; 800 ; 900) selon la revendication 2, dans lequel le bâti évasé (112) comprend une pluralité de segments de bâti en forme de pétale (112s).

4. Dispositif de centrage auto-expansible de capture d'emboles (400 ; 500 ; 600a ; 600b ; 600c ; 700 ; 800) selon l'une quelconque des revendications 1 à 3, dans lequel le matériau de filtre (119) est positionné sur les une ou plusieurs ouvertures (112o) dans la partie proximale (110p) de l'élément de centrage (110).

5. Dispositif de centrage auto-expansible de capture d'emboles (400 ; 500 ; 600a ; 600b ; 600c ; 700 ; 800) selon l'une quelconque des revendications 1 à 3, dans lequel le matériau de filtre (119) est positionné en aval des une ou plusieurs ouvertures (112o) dans la partie proximale (110p) de l'élément de centrage (110).

6. Dispositif de centrage auto-expansible de capture d'emboles (500 ; 600a ; 600b ; 600c ; 700 ; 800) selon la revendication 5, dans lequel le matériau de filtre (119) s'étend à partir d'une extrémité distale (110e) de l'élément de centrage (110), se plie sur et s'enroule, de manière proximale, autour de la surface externe (110s) de l'élément de centrage (110), et se fixe au dispositif (500 ; 600a ; 600b ; 600c ; 700 ; 800) dans une position à proximité des une ou plusieurs ouvertures (112o) dans la partie proximale (110p) de l'élément de centrage (110).

7. Dispositif de centrage auto-expansible de capture d'emboles (500 ; 600a ; 600b ; 600c ; 700 ; 800) selon la revendication 6, dans lequel l'élément de centrage (110) comprend un bâti évasé (112) et un matériau de recouvrement (114) disposé sur une partie distale du bâti évasé (112), dans lequel le matériau de recouvrement (114) est configuré pour bloquer le passage du sang à travers la matériau de recouvrement (114), et (i) dans lequel le matériau de filtre (119) est une extension du matériau de recouvrement (114) dans laquel des pores sont formés ou (ii) dans lequel le matériau de filtre (119) est un matériau qui est distinct du matériau de recouvrement (114).

8. Dispositif de centrage auto-expansible de capture d'emboles (600a ; 600b ; 600c) selon la revendication 5, comprenant une ou plusieurs poches de filtre (119) ayant une ou plusieurs bouches qui sont fixées sur l'élément de centrage (110) et capturent le fluide sortant des une ou plusieurs ouvertures dans la partie proximale (110p) de l'élément de centrage (110).

9. Dispositif de centrage auto-expansible de capture d'emboles (600b) selon la revendication 8, dans lequel chacune des une ou plusieurs poches de filtre (119) est fixée à une attache (117), ou dans lequel chacune des une ou plusieurs poches de filtre (119) est fixée à un élément qui coulisse le long d'un axe de l'arbre allongé (116), ou dans lequel chacune des une ou plusieurs poches de filtre (119) est fixée à l'arbre allongé (116).

10. Dispositif de centrage auto-expansible de capture d'emboles (700 ; 800) selon la revendication 5, dans lequel le matériau de filtre (119) est supporté par une structure de boucle expansible (115) qui subit une auto-expansion conjointement avec l'élément de centrage (110) et a un diamètre qui est supérieur à celui de l'élément de centrage (110) et est supporté dans une position longitudinale qui est approximativement la même que celle de l'extrémité distale ouverte (110o) de l'élément de centrage (110).

11. Dispositif de centrage auto-expansible de capture d'emboles (700) selon la revendication 10, dans lequel la structure de boucle expansible (115) est supportée par un ou plusieurs éléments structurels (115s) qui s'étendent entre la structure de boucle expansible (115) et l'extrémité distale de l'élément de centrage (110) ou dans lequel la structure de boucle expansible (115) est supportée par un ou plusieurs éléments structurels (115s) qui s'étendent entre la structure de boucle expansible (115) et l'arbre allongé (116).

12. Dispositif de centrage auto-expansible de capture d'emboles (700 ; 800) selon la revendication 10, dans lequel le matériau de filtre se présente sous la forme d'un sachet filtrant (119) ayant une bouche qui se fixe à la boucle (115) et à travers laquelle l'arbre allongé (116) passe dans une position à proximité de l'élément de centrage (110).

13. Dispositif de centrage auto-expansible de capture d'emboles (400 ; 500 ; 600a ; 600b ; 600c ; 700 ; 800) selon la revendication 5, dans lequel le matériau de filtre (119) est fixé à un bâti d'expansion qui est fixé à l'arbre allongé (116) dans une position à proximité de l'élément de centrage (110).

14. Dispositif de centrage auto-expansible de capture d'emboles (400 ; 500 ; 600a ; 600b ; 600c ; 700 ; 800) selon la revendication 13, dans lequel le bâti d'expansion a un diamètre supérieur à l'élément de centrage (110).

15. Système comprenant : (a) le dispositif de centrage auto-expansible de capture d'emboles (100, 400 ; 500 ; 600a ; 600b ; 600c ; 700 ; 800 ; 900) selon l'une quelconque des revendications 1 à 14, (b) un cathéter de guidage (120) ayant une lumière, et (c) un fil-guide (130), dans lequel le dispositif de centrage auto-expansible de capture d'emboles (100, 400 ; 500 ; 600a ; 600b ; 600c ; 700 ; 800 ; 900) est configuré pour être comprimé dans une configuration à profil bas pour l'avancement à travers la lumière du cathéter de guidage (120) et pour subir une auto-expansion dans une configuration expansée lorsqu'il est expulsé de la lumière du cathéter de guidage (120), et dans lequel le fil-guide (130) est configuré pour être avancé à travers une lumière de l'arbre allongé (116) du dispositif de centrage auto-expansible de capture d'emboles (100, 400 ; 500 ; 600a ; 600b ; 600c ; 700 ; 800 ; 900).
